# EUROPEAN PATENT SPECIFICATION

(11) **EP 1 805 305 B1**
(45) Date of publication and mention of the grant of the patent: **07.01.2009**
(21) Application number: 05795367.1
(22) Date of filing: 20.10.2005
(51) Int. Cl.: C12N 15/11, C12N 15/87, C12N 15/88

(54) **VEHICLE FOR THE TRANSPORT OF A CHOSEN MOLECULE TO A CELL**
TRÄGER FÜR DEN TRANSPORT EINES AUSGEWÄHLTEN MOLEKÜLS ZU EINER ZELLE
VÉHICULE DESTINÉ AU TRANSPORT D'UNE MOLÉCULE SÉLECTIONNÉE VERS UNE CELLULE

(30) Priority: 21.10.2004 NL 1027311; 04.11.2004 NL 1027417; 10.11.2004 NL 1027479
(43) Date of publication of application: 11.07.2007
(73) Proprietor: Synvolux IP B.V., 9713 GX Groningen (NL)
(72) Inventor: RUITERS, Marcel, Herman, Josef, NL-9728 WN Groningen (NL)
(74) Representative: Kupecz, Arpad
(86) International application number: PCT/NL2005/000754
(87) International publication number: WO 2006/043811

(56) References cited:
- EP-A- 0 755 924
- WO-A-93/04701
- WO-A-96/15811
- US-A- 5 958 894
- REJMAN J ET AL: "Characterization and transfection properties of lipoplexes stabilized with novel exchangeable polyethylene glycol-lipid conjugates" BIOCHIMICA ET BIOPHYSICA ACTA. BIOMEMBRANES, AMSTERDAM, NL, vol. 1660, no. 1-2, 28 January 2004 (2004-01-28), pages 41-52, XP004486900 ISSN: 0005-2736
- IRENE VAN DER WOUDE TE AL: "Novel pyridinium surfactants for efficient, nontoxic in vitro gene delivery" PROCEEDINGS OF THE NATIONAL ACADEMY OF SCIENCES OF USA, NATIONAL ACADEMY OF SCIENCE, WASHINGTON, DC, US, vol. 94, February 1997 (1997-02), pages 1160-1165, XP002094014 ISSN: 0027-8424
- MELTON ET AL.: "Optimization of small-scale coupling of A5B7 monoclonal antibody to carboxypeptidase G2" J. IMMUNOL. METHODS, vol. 158, no. 1, January 1993 (1993-01), pages 49-56,

## Description

The presented invention relates to a vehicle for the transport of a chosen molecule to a cell, comprising a SAINT-molecule, which by means of an electrostatic interaction, more particular a non-covalent binding, for instance by hydrogen bonding, is bound to the chosen molecule. Further the invention relates to the application of the SAINT-molecule for a targeted transport of a chosen molecule to a cell.

It is known in the state of the art to administer chosen molecules to a body, in such a way that the chosen molecule has to perform its action in or near the preferred cell of the body. One possibility to deliver such molecules to a cell is the administration of considerable amounts of chosen molecules to the body, whereby, by diffusion, a required concentration of the chosen molecule will be obtained at the targeted cells. A problem of this process is that the chosen molecule might generate undesirable site effects in other parts of the body.

REJMAN J ET AL: "Characterization and transfection properties of lipoplexes stabilized with novel exchangeable polyethylene glycol-lipid conjugates" BIOCHIMICA ET BIOPHYSICA ACTA. BIOMEMBRANES, AMSTERDAM, NL, vol. 1660, no. 1-2,28 January 2004 (2004-01-28), pages 41-52, XP004486900 ISSN: 0005-2736 discloses characterization and transfection properties of lipoplexes stabilized with novel exchangeable polyethylene glycol-lipid conjugates.

Therefore processes have been developed to deliver chosen molecules, for instance drugs, to a specific location in the body at a specific cell. A first method applied comprises the coupling of the chosen compound to antibodies. Such a coupling is covalent. As an effect, the activity of the chosen compound is substantially reduced in comparison with the activity of the free molecule. Another, currently applied method, is the packaging of the chosen molecules in liposomes. To the outside of the liposome, in which the molecule is packaged, an antibody can be coupled. This coupling of the antibody to the liposome occurs mostly after the inclusion of the chosen molecule in the liposome. In case the coupling of the antibody occurs before the chosen molecule has been included, it is possible that the antibody is located at the inner membrane of the liposome which might result in a decreased efficacy of the liposome. A general known disadvantage of inclusion of chosen molecules, for instance pharmaceutical compounds, in liposomes, is that pharmaceuticals will slowly leak from the liposome. A second disadvantage is that the formation of the packaging (the liposome containing the chosen compound inside) has to be performed in a solution containing the chosen compound. During packaging, not more than 5% of the chosen compounds will be included in the liposome. This means that 95% of the chosen compound is not included in the liposomes. Consequently, the efficacy of this method is very low.

The present invention aims at providing a vehicle which strongly improves the delivery of a chosen compound to a cell.

Furthermore the invention aims to provide a vehicle that enables long-lasting and stable binding to the chosen molecule.

To reach at least one of the aforementioned goals, the present invention provides a vehicle as mentioned in the preamble, characterised by the measures according to claim 1. In this way, a very stable process is provided to deliver a chosen molecule at a cell.

Although below hydrogen interaction and hydrogen bonding will be mentioned in general, the interaction is not limited to this single form. In all cases, any kind of electrostatic interaction is referred to.

In this text the word "cell" is used in general. With this, however, as the person skilled in the art will clearly understand, a "specific cell" is mentioned towards which the chosen compound has to be transported to.

Preferred embodiments are described in claims 2, 3 and 4.

According to another aspect, the invention relates to the application of a SAINT-molecule to transport a chosen molecule, in a targeted way, to a cell. Saint-molecule are commonly known, also described as transport vehicle, and are extensively described in the European patent, publication number EP-0755924 B1 and in the American patents with publication numbers US 5,853,694 and US 6,726,894, which is registered to the present applicant. Saint-molecules are, according to a broad description, to be regarded as synthetic amphiphiles.

The way chosen molecules, for instance macromolecules or pharmaceuticals in general, are enwrapped in SAINT-molecules, is described extensively in the aforementioned European patent EP 0755924 B1 and the American Patents US 5,853,694 and US 6,726,894.

According to a preferred embodiment, the SAINT-molecule is covalently bound to a linker molecule. By means of this covalently coupling it is assured that is an integrated and solid part of the stable vehicle. The linker molecules might be bound to the SAINT-molecules at the positions R1, R2, R3, R4, R5 and R5', taking into account that R5 and R5' either can be identical or different. For a more extended description of these type of SAINT-molecules reference is made to US 6,726,894. Examples of the linker molecules are for instance the following: 1-N'-Methyl-4-(aminobutyl, cis-9-oleyl)-methylpyrimidiniumchloride (SAINT-amino-linker), and 1-N'-methyl-4-(N-succinimidyl-S-acetylthio-acetate, cis-9-oleyl)-methylpyridiniumchloride (SAINT-SATA-linker). In these both cases the acetylthioacetate and the SATA group function as the linker(moiety). However equivalent compounds also might be applied as linker- moiety, including every chemical compound which can be covalently bound to SAINT-molecules. Although in general only one linker molecule will be bound to the SAINT-molecule, it might be possible that a combination of positions is occupied by a linker molecule, for example R1, R2, R3, R4, R5 or R5' alone or a combination of these, with a maximum off all five linker positions.

According to a further preferred embodiment, the cell specific ligand is chosen from: an antibody or a derivate thereof, a protein, a peptide, a compound with a specific application as a target for a cell surface, and other compounds with the desired cell specificity.

The antibodies or derivates thereof may be generated synthetically, or naturally occurring variants.

Within the scope of the present invention we consider as a ligand, every compound which is able to bind to a receptor or protein of a cell. A precondition for the ligand is that it has a specificity for a receptor and/or a cell type. Examples for these types of ligands are L-Dopa or adrenaline derivates. This first type of ligands specifically binds the dopamine receptors in the substantia nigra; the second type of ligand specifically binds to the adrenergic receptors of the body. Of course, the invention is not restricted to this example.

More specifically, it is preferred that the molecule to be enwrapped binds at the active surface of the SAINT-molecule. Without to be restricted to this embodiment, here the positively charged pyridiniumgroup is used. This group binds to the negative charge of the macromolecule to be enwrapped. Furthermore it is possible that the SAINT-molecules will bind the macromolecule in an indirect manner, by interacting with the water-layer surrounding the macromolecule, in case the macromolecule has a positive charge. Consequently, the SAINT-molecules will bind the macromolecules by means of an electrostatic interaction, for instance by forming hydrogen-bonds, so preventing leakage of the chosen molecules from the saint-molecules.

Furthermore the possibility exists that electrostatic interaction from, for instance, the carbon-atoms or the ortho-electrons of the pyridinium-ring, is for binding.

The present invention creates the possibility to deliver chosen molecules, for example a drug, at a specific place in the body at a specific cell. Hereby the advantage is generated that the amount of drugs, which has to been administered to the body, can be geared exactly for the amount of cells which needs to be treated. By choosing the appropriate formulation between the Saint-molecules and the enwrapped compound, leakage of the compound is prevented, and it is released after delivery to, fusion with, or uptake into the cell membrane, which also contains the receptor ligand that is complementary for the linker coupled ligand. In this way it is prevented that the chosen molecule is released at other places in the body. Here they stay included in the saint-molecule-vehicle.

### EXAMPLE

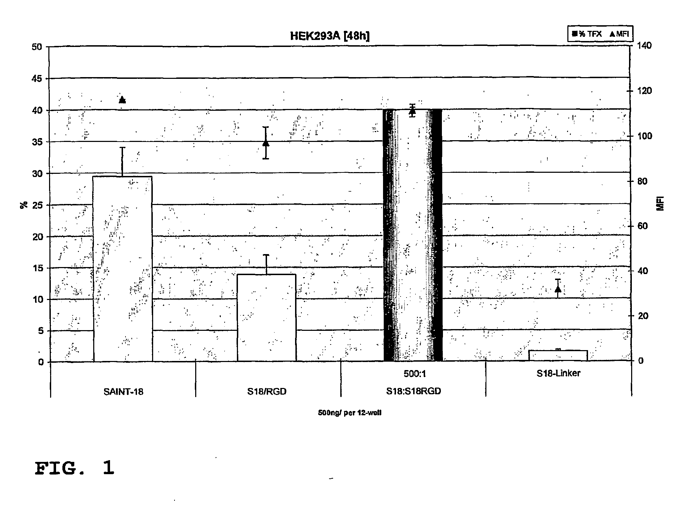

Human Embryonic Kidney cells are difficult to be transfected. When using these cells, main competing transfection methods (of other manufacturers), require several micrograms of plasmid DNA for successful transfection. Combined with SAINT-18, 1 µg (per 12 wells) normally results in a transfection rate above 90%. To be able to visualise the enhancing effect of the targeting moiety SAINT-18-RGD, in this protocol we used a low amount of plasmid DNA (500 ng per 12 wells). The structural formula of SAINT-linker-RGD is depicted in figure 1 below. In addition, an RGD-peptide is coupled to the SATA-group of the SAINT-Linker, as described in the present invention. The RGD group is a targeting moiety for the integrin receptor family and is though to increase the transfection efficacy. Human Embryonic Kidney cells, strain 293A, are cultured in 12 well plates and are transfected with 4 combinations of SAINT-molecules (i.e. SAINT-18, SAINT-18 coupled to RGD, a mixture of SAINT-18 and SAINT-18 coupled to RGD, and only a linker molecule as a reference). 500 ng of CMV-GFP plasmid is complex with 3.75nm SAINT molecules or linker (per well). 48 hours after transfection GFP expression is measured by FACS analysis. The percentage of GFP positive cells in depicted in the graph below.

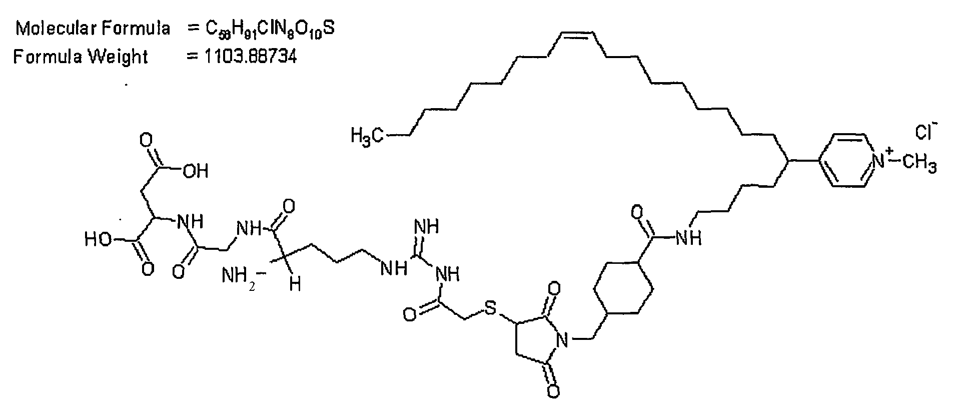

Structural formula of SAINT-linker-RGD.

The results depicted in Fig. 1 clearly show that
1. S18-linker (reference) functions poorly as a transfection reagent (column 4).
2. S18/RGD is able to transfect cells.
3. S18:S18RGD ratio 500:1 increases the transfection rate by approximately 33%.

S18-linker alone has no major transfection ability. We envision that 100% S18-RGD has a decreased transfection efficacy resulting from the sterical hindrance generated by the RGD group. However when S18RGD is mixed with S18 in a ratio of 500:1, transfection efficacy is greatly increased. A more optimised ratio will be determined.
Similar results are obtained with targeting moieties other than RGD (such as a cell-specific ligand). However, in all cases, an optimisation will be needed to gain an optimal effect. This experiment greatly indicates the transfection enhancing characteristic of Saint molecules.

## Claims

1. A vehicle for the transport of a chosen molecule to a cell, the constituent of which vehicle consists of one or more SAINT-linker molecules, which is **characterised in that** the SAINT-molecule is covalently bound to the linker molecule which is covalently bound to an antibody or a derivate thereof, a protein, a peptide, a compound that targets a cell-surface, or other compounds with cell specificity, and wherein said Saint-molecule is a synthetic amphiphile comprising a positively charged pyridiniumgroup.

2. The in vitro application of a SAINT-molecule for the targeted transport of a chosen molecule to a cell, in which the SAINT-molecule is bound to the chosen molecule by means of an electrostatic interaction, and in which the SAINT-molecule is covalently coupled to the linker molecule and the linker molecule is covalently coupled to the cell specific ligand, and wherein said SAINT-molecule is a synthetic amphiphile comprising a positively charged pyridiniumgroup,

3. The application according to claim 2, using a vehicle according to claim 1.

## Patentansprüche

1. Träger für den Transport eines gewählten Moleküls zu einer Zelle, wobei der Bestandteil dieses Trägers aus einem oder mehreren SAINT-Linker-Molekülen besteht, **dadurch gekennzeichnet, dass** das SAINT-Molekül an das Linker-Molekül kovalent gebunden ist, welches an einen Antikörper oder ein Derivat von diesem, ein Protein, ein Peptid, eine Verbindung, die auf eine Zelloberfläche zielgerichtet ist, oder weitere Verbindungen mit Zellspezifität kovalent gebunden ist, und wobei das SAINT-Molekül ein synthetisches Amphiphil ist, das eine positiv geladene Pyridiniumgruppe aufweist.

2. *In vitro* Verwendung eines SAINT-Moleküls für den zielgerichteten Transport eines gewählten Moleküls zu einer Zelle, wobei das SAINT-Molekül an das gewählte Molekül mittels einer elektrostatischen Wechselwirkung gebunden ist, und wobei das SAINT-Molekül an das Linker-Molekül kovalent gebunden ist und das Linker-Molekül an den zellspezifischen Liganden kovalent gebunden ist, und wobei das SAINT-Molekül ein synthetisches Amphiphil ist, das aus einer positiv geladenen Pyridiniumgruppe besteht.

3. Verwendung nach Anspruch 2, bei der ein Träger gemäß Anspruch 1 verwendet wird.

## Revendications

1. Véhicule pour le transport d'une molécule sélectionnée vers une cellule, le constituant dudit véhicule étant constitué d'une ou plusieurs molécules lieur-SAINT, **caractérisé en ce que** la molécule-SAINT est liée de façon covalente à la molécule lieur qui est liée de façon covalente à un anticorps ou un dérivé de celui-ci, une protéine, un peptide, un composé qui permet le ciblage à une surface cellulaire, ou d'autres composés ayant une spécificité cellulaire, et dans lequel ladite molécule-SAINT est une molécule amphiphile synthétique comprenant un groupe pyridinium chargé positivement.

2. Application *in vitro* d'une molécule-SAINT pour le transport ciblé d'une molécule sélectionnée vers une cellule, dans laquelle la molécule-SAINT est liée à la molécule sélectionnée par une interaction électrostatique, et dans laquelle la molécule-SAINT est liée de façon covalente à la molécule lieur et la molécule lieur est couplée de façon covalente au ligand ayant une spécificité cellulaire, et dans laquelle ladite molécule-SAINT est une molécule amphiphile synthétique comprenant un groupe pyridinium chargé positivement.

3. Application selon la revendication 2, utilisant un véhicule selon la revendication 1.
